**Europäisches Patentamt**

**European Patent Office**

**Office européen des brevets**

(19)

(11) **EP 1 256 360 A1**

(12) **DEMANDE DE BREVET EUROPEEN**

(43) Date de publication:
**13.11.2002 Bulletin 2002/46**

(51) Int Cl.$^7$: **A61P 1/12**, A61P 1/00,
A61P 39/06, A61K 33/42

(21) Numéro de dépôt: **01201715.8**

(22) Date de dépôt: **10.05.2001**

(84) Etats contractants désignés:
**AT BE CH CY DE DK ES FI FR GB GR IE IT LI LU
MC NL PT SE TR**
Etats d'extension désignés:
**AL LT LV MK RO SI**

(71) Demandeurs:
- **Université de Liège
  4020 Liège (BE)**
- **UNIVERSITE CATHOLIQUE DE LOUVAIN
  B-1348 Ottignies (Louvain la Neuve) (BE)**

(72) Inventeurs:
- **Gustin, Pascal, Prof, Université de Liège
  B-4000 Liège (BE)**
- **Cambier, Carole Dr., Université de Liège
  B-4000 Liège (BE)**
- **Clerbaux, Thierry Dr.,
  Univ. Catholique de Louvain
  B-1200 Bruxelles (BE)**
- **Frans, Albert Prof., Univ. Catholique de Louvain
  B-1200 Bruxelles (BE)**
- **Detry, M. Bruno, Univ. Catholique de Louvain
  B-1200 Bruxelles (BE)**

(54) **Composition médicamenteuse hypertonique et en particulier utilisation dans la fluidothérapie**

(57) Composition médicamenteuse comprenant, en quantités thérapeutiquement efficaces,du chlorure de sodium hypertonique,
au moins une molécule exerçant de manière directe ou indirecte un effet de relaxation des muscles lisses vasculaires, et
au moins une molécule susceptible de fournir un apport exogène en phosphates.

**EP 1 256 360 A1**

**Description**

La présente invention est relative à une nouvelle composition médicamenteuse, et en particulier à son utilisation dans la fluidothérapie.

Chez les bovins, les pathologies toxi-infectieuses, parmi lesquelles la diarrhée néonatale et le choc endotoxinique, nécessitent un tel traitement.

En ce qui concerne la diarrhée néonatale, il est communément admis que le premier traitement à instaurer est la fluidothérapie orale ou parentérale, en fonction de l'état clinique de l'animal. Cependant, les thérapeutiques actuelles visent uniquement à rétablir l'équilibre hydrique et ionique, à rétablir le pH sanguin et à couvrir un déficit énergétique éventuel. Jusqu'ici, le traitement habituellement conseillé dans la littérature scientifique traitant de la fluidothérapie parentérale chez le bovin repose sur l'administration de larges volumes de solutions cristalloïdes isotoniques, les quantités perfusées étant calculées en fonction notamment du degré de déshydratation et d'acidose de l'animal. Afin de rétablir le volume circulant, d'autres auteurs préconisent également l'administration de petits volumes d'une solution saline hypertonique, additionnée ou non de dextran. Selon la littérature, ces solutions salines doivent être accompagnées de l'administration d'un agent alcalinisant afin de rétablir le pH sanguin. Il apparaît donc qu'à ce jour, les troubles de l'oxygénation tissulaire existant chez le veau diarrhéique ne sont jamais pris en compte dans l'évaluation de l'efficacité du traitement. Plus grave, dans certains cas, les thérapeutiques actuelles peuvent même aggraver ces troubles de l'oxygénation tissulaire. En effet, par exemple, l'administration massive de bicarbonates en vue de rétablir le pH peut causer une alcalose relative et une hypochlorémie, favorisant ainsi l'augmentation de l'affinité de l'hémoglobine pour l'oxygène et rendant le transport de l'oxygène par le sang moins efficient.

En ce qui concerne le choc endotoxinique, il est également admis que l'instauration d'un traitement par fluidothérapie parentérale est une priorité. Cependant, celui-ci vise essentiellement à rétablir le volume circulant. La littérature scientifique décrit l'utilisation de différentes solutions de perfusion, à savoir : les solutés cristalloïdes isotoniques, les solutés cristalloïdes hypertoniques (additionnés ou non d'un colloïde comme le dextran), mais aussi le plasma et le sang total. Les effets de ces différentes thérapeutiques sur le transport de l'oxygène par l'hémoglobine et l'oxygénation des tissus n'ont toutefois pas été étudiés de manière approfondie.

Par ailleurs, les brevets US 5.238.684, 5.236.712, 5.147.650, 5.089.477 et 4.981.687 décrivent des formulations destinées à être administrées de préférence par voie orale, qui visent à augmenter la réponse physiologique à l'exercice, notamment via une augmentation du débit cardiaque. Ces solutions contiennent de l'eau, des électrolytes, du glycérol et une source d'énergie additionnelle. Ces solutions ne visent aucunement à manipuler le transport de l'oxygène par l'hémoglobine, ni à améliorer l'oxygénation tissulaire.

La présente invention a pour but de mettre au point une nouvelle composition médicamenteuse. Celle-ci doit pouvoir lutter contre l'hypoxie tissulaire qui survient au cours de pathologies toxi-infectieuses, chez les mammifères, en particulier chez les ruminants, tels que les bovins, et y compris les êtres humains. Au cours de ces processus pathologiques, l'invention doit permettre de maintenir chez les êtres atteints leur consommation en oxygène grâce, notamment, à une augmentation de la quantité d'oxygène extraite au niveau des tissus, tout en combinant ce mécanisme original avec une augmentation du débit cardiaque.

Pour résoudre ce problème, il est prévu, suivant l'invention, une composition médicamenteuse comprenant, en quantités thérapeutiquement efficaces, du chlorure de sodium hypertonique, au moins une molécule exerçant de manière directe ou indirecte un effet de relaxation des muscles lisses vasculaires, et au moins une molécule susceptible de fournir un apport exogène en phosphates. Cette composition comprend un "trépied" thérapeutique nécessaire pour garantir le succès thérapeutique visé. Elle agit par une combinaison de mécanismes d'action originaux permettant une meilleure extraction de l'oxygène au niveau tissulaire, à savoir une diminution de l'affinité intrinsèque de l'hémoglobine pour l'oxygène, une augmentation in vivo du transport de l'oxygène par l'hémoglobine et une augmentation de la capacité intrinsèque des tissus à extraire l'oxygène. La meilleure extraction de l'oxygène au niveau tissulaire évite le développement d'une hypoxie tissulaire.

Suivant une forme de réalisation de l'invention, la composition comprend en outre un agent alcalinisant susceptible d'augmenter un pH sanguin dans une gamme compatible avec un transport d'oxygène adéquat. Cet agent alcalinisant en quantité adéquate permet un maintien de l'acidose relative. Comme agents alcalinisants on peut mentionner entre autres des bicarbonates, des acétates, des propionates et des lactates, en particulier du bicarbonate de sodium.

Suivant une forme avantageuse de l'invention, la composition consiste en une combinaison d'une première solution de perfusion et d'une deuxième solution de perfusion, la première solution de perfusion comprenant de l'eau, ledit chlorure de sodium hypertonique et ladite au moins une molécule exerçant un effet de relaxation, et la deuxième solution de perfusion comprenant de l'eau, et ladite au moins une molécule susceptible de fournir un apport exogène en phosphates. Grâce à cette forme de réalisation, administrée par perfusion, par exemple par voie intraveineuse, il est possible d'augmenter le débit cardiaque ce qui renforce le phénomène d'extraction de l'oxygène au niveau tissulaire.

Une telle formulation permet une fabrication et un stockage séparés des composants ainsi qu'éventuellement une administration chronologiquement séparée des deux solutions, en fonction de la vitesse d'action des composants envisagés. Elle permet d'ailleurs aussi, si nécessaire, une perfusion simultanée des deux solutions à deux endroits différents du corps à traiter et une application étalée dans le temps de ces solutions.

Suivant l'invention, le chlorure de sodium est à une concentration de 5 à 10 % dans ladite première solution de perfusion. Avantageusement, on fera usage de doses d'environ 5 à 10 ml/kg de poids de corps. Par molécule exerçant un effet de relaxation des muscles lisses on peut entendre, suivant l'invention, les vasodilatateurs en général, et la caféine en particulier. Par molécule susceptible de fournir un apport exogène en phosphates, on peut entendre suivant l'invention un phosphate inorganique, par exemple du phosphate de sodium, ou un phosphate organique, tel qu'un triphosphate, par exemple du triphosphate d'adénosine, ou du diphosphoglycérate, ainsi qu'en général toute substance capable de modifier la teneur intra-érythrocytaire en diphosphoglycérate.

Suivant une forme particulière de l'invention, la deuxième solution de perfusion contient en outre au moins un composant destiné à corriger l'équilibre hydrique, électrolytique et énergétique. Par composant de ce genre, on peut entendre du chlorure de sodium, du chlorure de potassium, du glucose, etc.

D'autres compositions médicamenteuses suivant l'invention sont indiquées dans les revendications données dans la suite, ainsi que l'utilisation de ces compositions pour la fabrication d'un médicament destiné au traitement thérapeutique ou prophylactique de troubles hypoxiques des tissus, en particulier de ceux survenant au cours de pathologies toxi-infectieuses, chez les mammifères.

L'invention va à présent être décrite de manière détaillée à l'aide d'exemples donnés ci-dessous à titre non limitatif.

### Exemple 1

Dans les conditions normales, le transport de l'oxygène sous forme dissoute ne représente qu'une faible proportion (environ 1 %) du transport total. Pour le reste, l'oxygène est acheminé vers les tissus après fixation sur l'hémoglobine.

Au niveau pulmonaire, l'hémoglobine se sature en oxygène. Dans les tissus au repos, environ un tiers de la quantité totale ainsi fixée diffuse dans le liquide interstitiel, le reste retournant vers les poumons. La fixation de l'oxygène sur l'hémoglobine dépend avant tout de la pression partielle en oxygène ($PO_2$) dans le plasma.

Classiquement, chez tous les mammifères, les facteurs régulateurs de l'affinité de l'oxygène pour l'hémoglobine sont le pH sanguin, la température corporelle, la pression partielle en dioxyde de carbone, le taux de fixation de 2,3-diphosphoglycérate (2,3 DPG) sur l'hémoglobine.

Ces mécanismes permettent les échanges de l'oxygène dans l'organisme. Dans le sang artériel, la pression partielle en oxygène est élevée, la température et les teneurs en $CO_2$ diminuent, tandis que le pH augmente, autant de conditions favorables à la fixation de l'oxygène sur l'hémoglobine. Dans le sang veineux et dans les tissus, au contraire, la pression partielle en oxygène est faible, le pH diminue, la température et les teneurs en $CO_2$ augmentent, autant de facteurs favorables à la libération de l'oxygène.

Lors d'une étude ayant pour but d'investiguer les troubles de l'oxygénation tissulaire chez le veau diarrhéique, en considérant l'organisme non plus dans sa globalité, mais dans un territoire vital corporel en particulier, il a pu être démontré qu'une adaptation de cette oxygénation par une augmentation de l'extraction de l'oxygène au niveau tissulaire n'existait pas dans tous les organes. La capacité des tissus à extraire l'oxygène est même apparue diminuée. Il est apparu que, si l'on considère les échanges se produisant entre le sang artériel et le sang veineux jugulaire, les veaux diarrhéiques souffrent d'une incapacité à extraire l'oxygène qui leur est apporté par le sang. Cette incapacité peut être expliquée par :

- une augmentation de l'affinité de l'oxygène pour l'hémoglobine, illustrée par la diminution de la pression partielle en oxygène à 50 % de saturation de l'hémoglobine mesurée dans des conditions standards (pH : 7,4; $PCO_2$ : 40 mm de Hg; température : 37°C), appelée ci-après P50 standard (P50 std). Ce phénomène s'explique notamment par une diminution du taux de 2,3 DPG;

- une hypothermie et une hypocapnie, qui s'opposent aux effets positifs de l'acidose sur le transport de l'oxygène par le sang, ainsi que l'illustre la chute des pressions partielles en oxygène à 50 % de saturation de l'hémoglobine dans les compartiments artériel (P50a) et veineux (P50v);

- une incapacité intrinsèque des tissus à extraire l'oxygène qui leur est apporté, ainsi que l'illustre l'augmentation de la pression partielle en oxygène au niveau veineux ($PvO_2$) chez les veaux diarrhéiques.

On a par conséquent procédé dans le présent exemple à une série de traitements de veaux diarrhéiques par une méthode classique, par un produit du commerce, par différents composants d'une composition suivant l'invention et par une composition suivant l'invention, dans des buts de comparaison.

Dans tous les exemples ci-dessous, les dosages sont calculés par rapport à 1 kg de poids de corps.

a) La solution classique testée est une solution cristalloïde isotonique de chlorure de sodium (40 ml/kg), de glucose (20 ml/kg) et de bicarbonate de sodium, à perfuser. La quantité de bicarbonate de sodium à apporter est calculée comme suit: nombre de mmoles de bicarbonate à apporter = Poids (kg) x déficit en base dans le sang veineux (mmoles/l) x 0,6 (l/kg), où 0,6 représente le volume de distribution du bicarbonate dans le compartiment liquidien extracellulaire. Vitesse de perfusion: 50 ml/kg/h la première heure, puis 20 ml/kg/h.

b) La solution commerciale, le Lactétrol$^R$, est un médicament indiqué pour la correction des déshydratations, accompagnées ou non d'acidose métabolique, chez les bovins. Il est important de noter que ce produit est un des rares médicaments disponibles sur le marché pour le traitement, par fluidothérapie parentérale, de symptômes associés à la diarrhée néonatale. La formule de la spécialité est la suivante :

| | |
|---|---|
| Chlorure de sodium | 5,76 mg |
| Chlorure de potassium | 0,37 mg |
| Chlorure de calcium | 0,37 mg |
| Chlorure de magnésium | 0,2 mg |
| Lactate de sodium | 5,04 mg |
| Parahydroxybenzoate de méthyle | |
| Eau pour injection | ad 1 ml. |

Volume à administrer : 40 ml/kg. Vitesse de perfusion : 10 ml/kg/h.

c) Une composition constituée de deux solutions de perfusion :

- solution A hypertonique (NaCl 7,5 %, 5 ml/kg, 1 ml/kg/min) : cette solution étant à la limite de l'osmolarité permise en thérapeutique, les éléments visant à rétablir l'équilibre ionique et à maintenir l'équilibre hydrique et le pH sont amenés via une solution B, administrée séparément.
- solution B isotonique (glucose : 20 ml/kg et bicarbonate de sodium administré en quantité suffisante pour ramener le déficit en base à zéro). Cette solution peut éventuellement être complétée par d'autres ions (K$^+$, Mg$^{++}$ ...). Vitesse d'administration : 50 ml/kg/h la première heure, puis 20 ml/kg/h.

La quantité de bicarbonate de sodium à apporter est calculée comme précédemment décrit.

d) Une composition constituée de deux solutions de perfusion :

solution A hypertonique (NaCl 7,5 %, 7,5 ml/kg additionnée de 0,4 g de caféine par litre; 1 ml/kg/min) : cette solution étant à la limite de l'osmolarité permise en thérapeutique, les éléments visant à rétablir l'équilibre ionique et à maintenir l'équilibre hydrique et le pH sont amenés séparément via une solution B.
solution B (NaCl isotonique + glucose: 10 g/l + chlorure de potassium : 1 g/l : 40 ml/kg et bicarbonate de sodium isotonique administré en quantité suffisante pour ramener le pH à 7,2). Cette solution peut éventuellement être complétée par d'autres ions (Mg$^{++}$...). Vitesse d'administration : 20 ml/kg/h.

La quantité de bicarbonate de sodium à apporter a été calculée selon l'équation Henderson-Hasselbalch.

e) Une composition constituée comme suit :

chlorure de sodium isotonique + glucose: 10 g/l + chlorure de potassium : 1 g/l: 40 ml/kg, et phosphates inorganiques isotoniques (NaH$_2$PO$_4$.H$_2$O: 2,45 g/l, Na$_2$HPO$_4$.2H$_2$O: 19,82 g/l): 16 ml/kg. Cette solution peut éventuellement être complétée par d'autres ions (Mg$^{++}$...). Vitesse d'administration : 27,5 ml/kg/h.

f) Composition suivant l'invention constituée de deux solutions de perfusion :

solution A :

Eau stérile apyrogène
NaCl hypertonique (7,5 %) : 75 g/l
Caféine : 0,4 g/l
(7,5 ml/kg; vitesse de perfusion : 1 ml/kg/min)

solution B :

Eau stérile apyrogène.

Solution isotonique tampon de phosphates inorganiques ($NaH_2PO_4.H_2O$ : 2,45 g/l, $Na_2HPO_4.2H_2O$: 19,82 g/l) : 16 ml/kg; vitesse de perfusion : 25 ml/kg/h.

En vue de rétablir la volémie, de corriger l'équilibre électrolytique et de maintenir le pH dans des limites compatibles avec le transport de l'oxygène (7,2), divers composés peuvent être ajoutés à cette solution, à savoir : NaCl, KCl, $MgCl_2$, glucose, $NaHCO_3$.

Par exemple: solution de chlorure de sodium isotonique + glucose 10 g/l + chlorure de potassium 1 g/l (40 ml/kg) et bicarbonate de sodium isotonique si nécessaire (pH < 7,2).

Chacune de ces compositions a été testée sur un échantillon d'environ 10 veaux diarrhéiques. Il faut noter que dans le cas des compositions formées de deux solutions, la solution B a été perfusée 15 minutes après le début de la perfusion de la solution A.

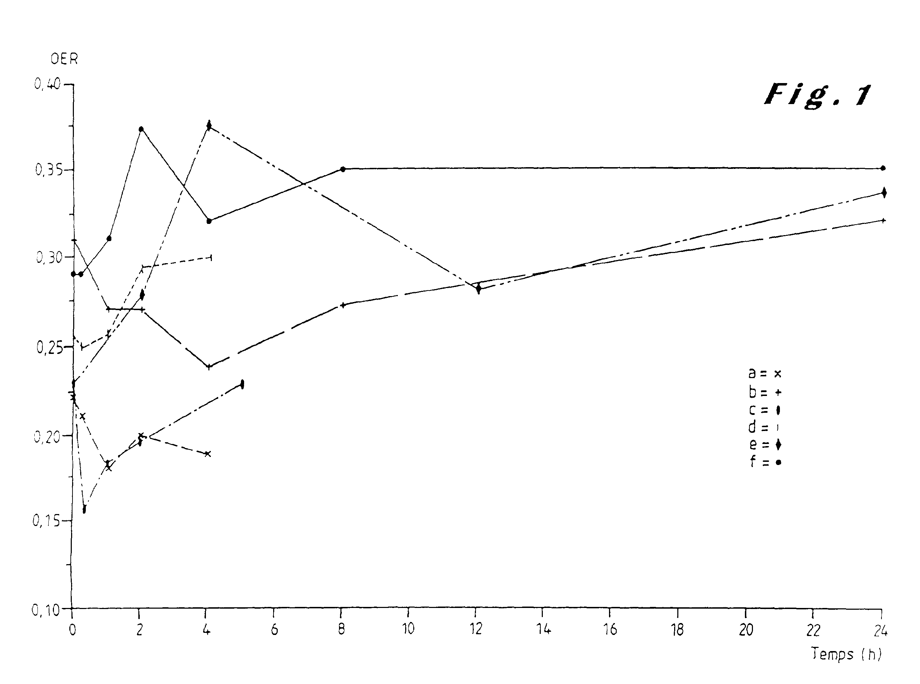

La figure 1 illustre l'évolution en fonction du temps de la quantité d'oxygène extraite au niveau tissulaire (OER) suite à l'administration de chacune des compositions a) à f). OER est le rapport entre la différence du contenu en oxygène dans le sang artériel ($CaO_2$) et veineux ($CvO_2$) et le contenu en oxygène dans le sang artériel. Pratiquement, le contenu artériel et le contenu veineux en oxygène sont calculés comme suit :

$$CaO_2 = Hb \times BO_2 \times [SaO_2/100] + (\alpha \times PaO_2)$$

$$CvO_2 = Hb \times BO_2 \times [SvO_2/100] + (\alpha \times PvO_2)$$

où Hb représente le taux d'hémoglobine de l'animal, $BO_2$ représente la quantité d'oxygène fixée par l'hémoglobine (1,39 $mlO_2$/g Hb) et $\alpha$ représente le coefficient de solubilité de l'oxygène (0,003 ml.100$ml^{-1}$ mm $Hg^{-1}$).

La fraction d'oxygène extraite au niveau tissulaire s'exprime alors comme un rapport : OER = ($CaO_2$ - $CvO_2$) /$CaO_2$

De cette figure 1 on peut déduire que le traitement classique a) et la solution Lactétrol b) ne sont pas capables d'augmenter l'extraction de l'oxygène au niveau tissulaire.

Alors que l'on avait remarqué un effet favorable de l'ion chlore sur le transport de l'oxygène in vivo, chez des veaux sains (Cambier et al, Effects of hyperchloremia on blood oxygen binding in healthy calves, The American Physiological Society, 1998, p. 1267-1272), ce traitement (composition c)) s'est malheureusement révélé incapable d'augmenter l'extraction de l'oxygène au niveau tissulaire des veaux diarrhéiques, ce paramètre ayant même tendance à diminuer. Les résultats obtenus, in vivo, chez le veau sain, ne sont donc pas transposables au veau malade.

Enfin, il apparaît avec la composition d) une légère augmentation de la quantité d'oxygène extraite, qui toutefois ne s'avère pas significative, tandis que la composition e) et la composition suivant l'invention f) présentent une augmentation significative et durable de celle-ci.

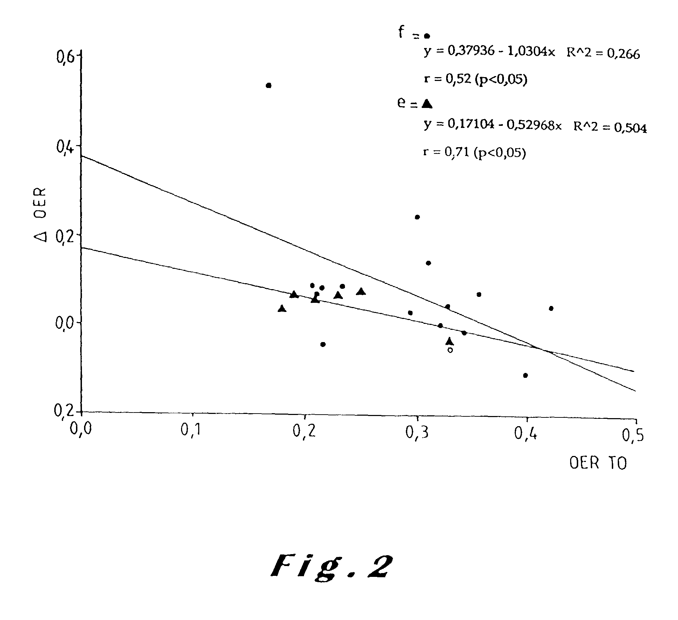

Cependant, l'avantage de la composition suivant l'invention f) par rapport à la composition e) apparaît lorsque les variations de l'OER enregistrées en cours de traitement sont mises en relation avec les valeurs initiales d'OER mesurées à T0 (figure 2). Une corrélation négative significative est observée avec les deux traitements. Ceci signifie que les animaux présentant les déficiences les plus marquées du point de vue de l'OER tirent le plus grand bénéfice des thérapies. Toutefois, comparant les deux solutions e) et f), il apparaît que les augmentations d'OER obtenues avec la composition e) sont inférieures à celles obtenues avec la composition suivant l'invention f), ainsi qu'en attestent les ordonnées à l'origine des équations des régressions linéaires relatives à ces deux traitements (respectivement 0,17 pour la composition e) et 0,38 pour la composition suivant l'invention f).

En outre, il faut souligner que seule la composition suivant l'invention f) met en jeu tous les mécanismes permettant une augmentation de l'extraction de l'oxygène au niveau tissulaire. Ce phénomène peut se révéler très important en conditions pathologiques, un mécanisme pouvant suppléer l'autre en cas de déficience.

Pour rappel, l'extraction de l'oxygène au niveau tissulaire peut être améliorée via trois mécanismes :

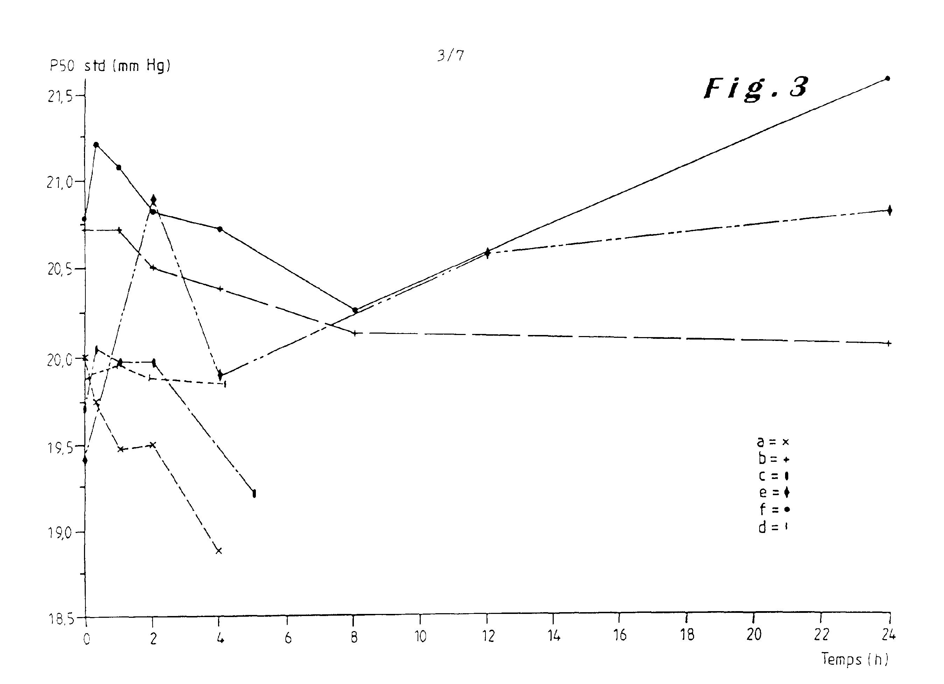

1. par une diminution de l'affinité intrinsèque de l'hémoglobine pour l'oxygène. Ce phénomène est illustré par une augmentation de la P50 std. La figure 3 montre une augmentation significative de la P50 std lors de l'administration de la composition e) et lors de l'administration de la composition suivant l'invention f) alors qu'aucun effet significatif n'est obtenu, ou même que des effets délétères (diminution de la P50 standard) sont enregistrés, avec les autres traitements.

2. par une modification, in vivo, des facteurs de contrôle du transport de l'oxygène par le sang, et notamment le pH. Les effets bénéfiques enregistrés, in vitro, sur la P50 std (cf point 1) peuvent ainsi être augmentés ou diminués chez l'animal. In vivo, la capacité de transport de l'oxygène par le sang est évaluée en calculant les pressions partielles en oxygène P50 a et P50 v. Idéalement, une augmentation de la P50 a et de la P50 v est souhaitée, en renforçant, via notamment des variations de pH, les effets sur la P50 std.

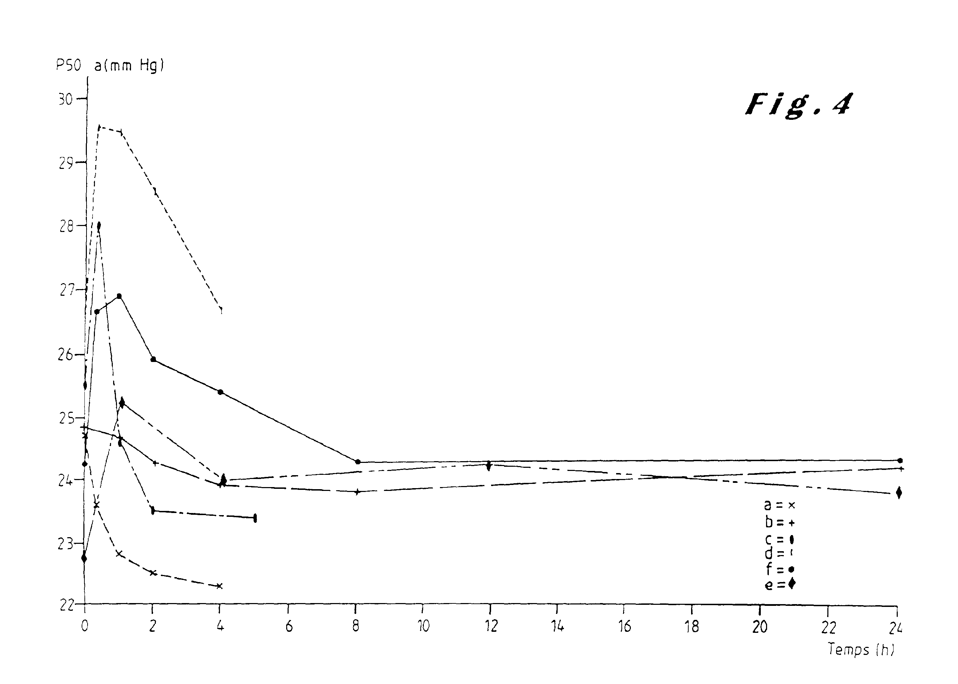

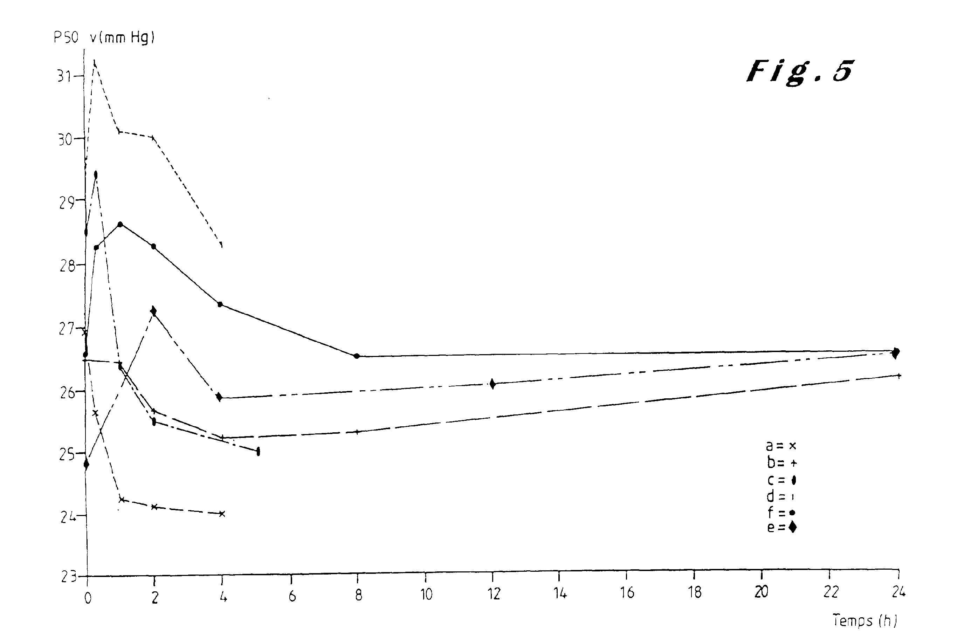

Comme l'illustrent les figures 4 et 5, in vivo, la composition e) et la composition suivant l'invention f), améliorent les capacités de transport de l'oxygène. Cependant, en ce qui concerne la composition e), cet effet trouve principalement son origine dans la diminution de l'affinité intrinsèque de l'hémoglobine pour l'oxygène, illustrée par l'augmentation de la P50 std. Ce phénomène n'est que peu renforcé in vivo. Ainsi, à t= 2 heures, si la composition e) augmente la P50 std de 8 %, les P50 a et P50 v ne sont augmentées que de, respectivement, 10 et 9 %, montrant l'absence d'intervention d'autres mécanismes favorables à la libération de l'oxygène au niveau tissulaire. In vivo, l'intervention de ces mécanismes régulateurs du transport de l'oxygène peut se révéler très importante chez des animaux dont la P50 standard n'augmente que faiblement suite à la perfusion. Ainsi les effets obtenus sur la P50 standard avec la composition e) sont variables. Pour preuve, à t= 2 heures, l'augmentation de P50 standard varie de 4 à 13 % selon les individus, et seuls 33 % des animaux traités avec la composition e) maintiennent une augmentation de P50 standard durant 24 heures. Chez ces animaux, le recours à d'autres mécanismes régulateurs serait nécessaire. Contrairement à la solution e), la composition suivant l'invention f) permet, outre l'augmentation de la P50 standard, le développement d'une hyperchlorémie et le maintien d'une acidose relative, deux éléments renforçant les effets sur la P50 standard et favorables à la libération de l'oxygène au niveau tissulaire. Le poids de ces divers mécanismes varie au cours du temps. Ainsi, à t = 1 heure, la composition suivant l'invention f) induit une augmentation de la P50 standard de seulement 1 % mais augmente les P50 a et P50 v, respectivement de 11 et de 8 %, du fait du développement d'une hyperchlorémie et du maintien d'une acidose relative. A t= 24 heures, la balance entre ces deux fonctions s'inverse, puisque le rôle joué par les modifications de l'affinité intrinsèque de l'hémoglobine pour l'oxygène devient prépondérant;

3. par une augmentation de la capacité des tissus à extraire l'oxygène, marquée par une diminution de la pression partielle en oxygène au niveau veineux ($PvO_2$).

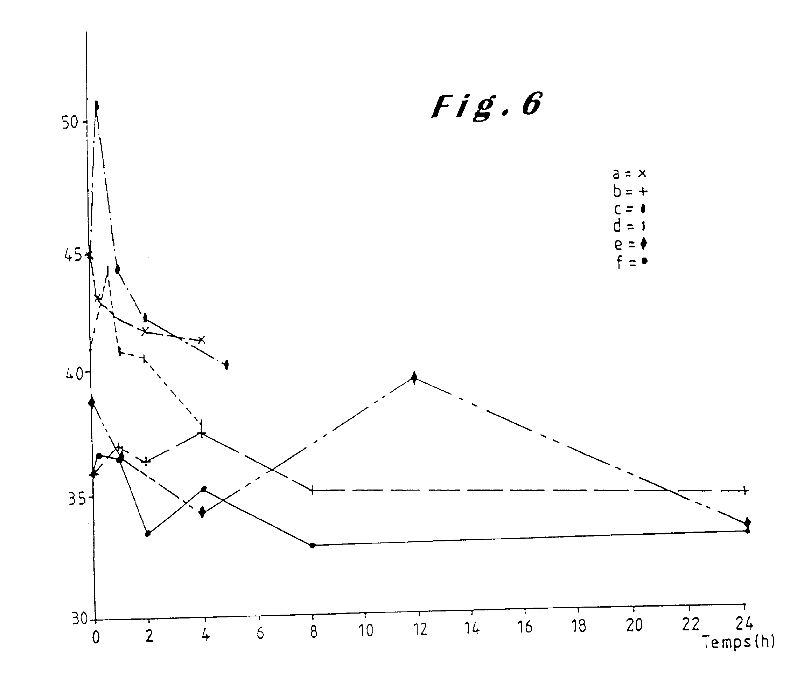

Ainsi que l'illustre la figure 6, la composition hypertonique c) augmente de manière significative la $PvO_2$. L'administration d'un vasodilatateur, simultanément à une solution hypertonique (composition d) ou l'absence de solution hypertonique, comme c'est le cas de la composition e), permet d'annuler cet effet. En combinant un vasodilatateur, une solution hypertonique de NaCl et les phosphates, selon la composition suivant l'invention f), la capacité propre des tissus à extraire l'oxygène est optimale et se maintient tout au long du traitement.

Les méthodes de détermination de la $PvO_2$ et des P50 sont décrites par exemple dans C. Cambier et al., American Journal of Veterinary Research, 61, 2000, p. 299-304.

Outre ces trois mécanismes intervenant dans l'augmentation de l'extraction de l'oxygène au niveau tissulaire, une solution de perfusion peut également induire des effets bénéfiques via des modifications hémodynamiques (augmentation du débit cardiaque).

Selon les données de la littérature, et sur base de notre propre expérience chez le veau atteint de choc endotoxinique (cf exemple 3), l'hypoxie peut résulter :

- d'une altération de l'apport d'oxygène par le sang ($DO_2$) consécutive, notamment, à des perturbations hémodynamiques (diminution du débit cardiaque),
- d'une diminution de l'extraction de l'oxygène au niveau tissulaire, qui vient d'être discutée.

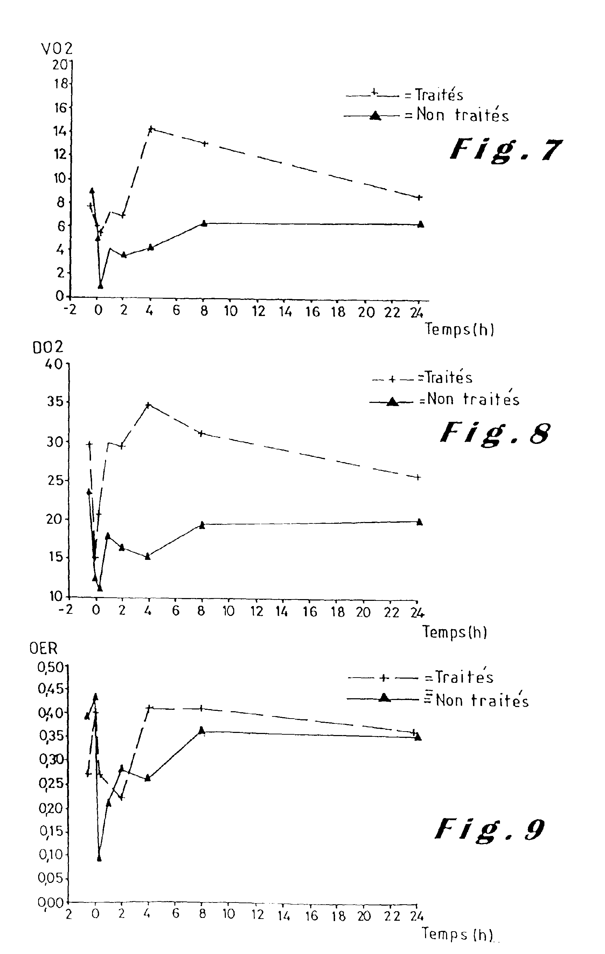

Ces deux mécanismes se combinent à des degrés divers chez les individus atteints de pathologies toxi-infectieuses pour aboutir aux état hypoxiques constatés cliniquement. Il est dès lors capital de disposer de solutions thérapeutiques permettant d'améliorer l'ensemble de ces fonctions, et ce par tous les mécanismes possibles. La solution suivant l'invention, outre ses effets sur l'OER, améliore la $DO_2$ du fait de l'augmentation du débit cardiaque liée à l'apport hydrique mais surtout à un effet hyperosmotique dans le compartiment intravasculaire. La figure 8 illustre l'augmentation de la $DO_2$ enregistrée chez des veaux souffrant de choc endotoxinique et traités avec la composition suivant l'invention.

L'administration d'une solution isotonique, comme la solution e), peut induire une augmentation du débit cardiaque du fait de l'apport hydrique mais cet effet reste limité et différé dans le temps par rapport aux effets quasiment immédiats et importants des solutions hypertoniques, comme la composition suivant l'invention f).

En résumé, outre le fait d'exercer un effet original plus important sur l'OER (cf figure 2) que la solution e), la solution suivant l'invention f) agit donc sur tous les mécanismes susceptibles de lutter contre les troubles de l'extraction de l'oxygène au niveau tissulaire.

De plus, elle exerce des effets hémodynamiques immédiats et importants de par ses effets directs mais aussi hyperosmotiques.

## Exemple 2

Un essai comparatif a été effectué sur trois échantillons de veaux diarrhéiques. Le premier échantillon (n = 12) a été soumis au traitement classique a) décrit dans l'exemple 1, le deuxième échantillon (n = 18) au Lactétrol et le troisième échantillon (n = 16) à la composition suivant l'invention f) de l'exemple 1.

On a ensuite établi un score clinique des veaux traités en analysant les symptômes de la maladie, et en intégrant des évaluations telles que la diminution du réflexe de succion, le ralentissement du réflexe à la menace, la diminution de la sensibilité tactile, la diminution de la capacité à se tenir debout, etc... Le score est de 0 pour les veaux sains et de 13 chez un animal comateux. Les résultats de cette évaluation sont réunis dans le tableau 1 ci-dessous.

Tableau 1

| Paramètres/Temps (h) | Invention (n=16) | Trt classique (n=12) | Trt lactétrol (n=18) |
|---|---|---|---|
| Score clinique | | | |
| 0 | $3,6 \pm 0,6$ | $5,1 \pm 1,2$ | $4,6 \pm 0,8$ |
| 0,25 | $3,3 \pm 0,5$ | $4,9 \pm 1,2$ | N.D. |
| 1 | $2,6 \pm 0,4^{**}\dagger\Delta$ | $4,9 \pm 1,1$ | $4,5 \pm 0,8$ |
| 2 | $1,7 \pm 0,3^{***}\dagger\Delta$ | $4,3 \pm 1,1$ | $3,9 \pm 0,7^{*}$ |
| 4 | $1,3 \pm 0,3^{***}\dagger\Delta\Delta$ | $3,3 \pm 0,9^{*}$ | $3,6 \pm 0,8^{**}$ |
| 8 | $0,9 \pm 0,2^{**}\Delta\Delta$ | N.D. | $2,9 \pm 0,6^{**}$ |
| 24 | $1,4 \pm 0,3^{***}\Delta$ | N.D. | $3,6 \pm 0,8^{*}$ |
| N.D. : non déterminé.        Trt : traitement | | | |
| Les valeurs sont exprimées comme suit : moyennes $\pm$ SE. | | | |
| * : valeur significativement différente de la valeur enregistrée à T0 dans le même groupe (* : p<0,05, ** : p<0,01, *** : p<0,001) | | | |
| † : valeur significativement différente de la valeur enregistrée au même moment dans le groupe traitement classique († : p<0,05) | | | |
| $\Delta$ : valeur significativement différente de la valeur enregistrée au même moment dans le groupe traitement lactétrol[R] ($\Delta$ : p<0,05, $\Delta\Delta$ : p<0,01). | | | |

## Exemple 3

Des troubles systémiques d'extraction de l'oxygène au niveau tissulaire ayant été mis en évidence lors de choc endotoxinique, un essai comparatif a été effectué sur deux échantillons de veaux sains ayant reçu une injection intraveineuse d'endotoxines d'Escherichia coli sérotype 055:B5, 0,2 $\mu$g/kg, au temps -30 minutes. Au temps 0, un premier échantillon (n = 3) est soumis au traitement d'une composition suivant l'invention et un deuxième échantillon (n = 3) n'est soumis à aucun traitement.

Ainsi que le montre la figure 7, contrairement à ce qui a été observé chez les animaux non traités, la composition suivant l'invention permet de maintenir la consommation en oxygène ($VO_2$) à son niveau de base, voire de l'augmenter, empêchant ainsi le développement d'une hypoxie tissulaire. Ce maintien, voire cette augmentation, sont rendus possibles grâce aux mécanismes suivants :

- un maintien de la quantité d'oxygène amenée aux tissus par le sang ($DO_2$), du fait notamment, de l'augmentation du débit cardiaque (effet hémodynamique). L'évolution de la $DO_2$ est reprise à la figure 8;
- une augmentation de la capacité d'extraction de l'oxygène au niveau tissulaire, illustrée à la figure 9.

Cette augmentation de l'OER s'explique par les mêmes phénomènes que ceux décrits chez les veaux diarrhéiques.

## Exemple 4

Un essai comparatif a été effectué selon la méthodologie décrite pour l'exemple 3. Au cours de cet essai, un score clinique a été établi selon les critères décrits pour l'exemple 2.

Les résultats de cette évaluation sont réunis dans le tableau 2 ci-dessous.

Tableau 2

| Paramètres/Temps (h) | Veaux traités (n=3) | Veaux non traités (n=3) |
|---|---|---|
| Score clinique | | |
| -0,5 | 1,0 ± 0,0 | 1,0 ± 0,0 |
| 0 | 4,3 ± 2,0 | 4,0 ± 0,6* |
| 0,25 | 4,7 ± 1,7* | 7,0 ± 0,3**Δ |
| 1 | 4,7 ± 1,7 | 7,7 ± 0,9*Δ |
| 2 | 3,8 ± 1,6 | 6,0 ± 0,6* |
| 4 | 2,5 ± 0,5* | 3,3 ± 0,3* |
| 8 | 1,17 ± 0,2 | 1,7 ± 0,7 |
| 24 | 1,0 ± 0,0 | 1,7 ± 0,7 |

Les valeurs sont exprimées comme suit : moyennes ± SE.

\* : valeur significativement différente de la valeur enregistrée à T-30 (valeur physiologique) dans le même groupe (\* : $p < 0,05$, \*\* : $p < 0,01$)

Δ: valeur significativement différente de la valeur enregistrée T0 dans le même groupe (Δ : $p < 0,05$).

Il doit être entendu que la présente invention n'est en aucune façon limitée aux formes de réalisation décrites ci-dessus et que bien des modifications peuvent y être apportées sans sortir du cadre des revendications annexées.
On peut par exemple envisager une composition sous la forme d'au moins une formulation solide, par exemple une poudre ou un comprimé soluble ou effervescent, destinée à la constitution d'au moins une solution aqueuse de perfusion par mélange avec de l'eau. Ainsi, on peut aisément stocker les composants de la composition suivant l'invention et préparer les solutions à perfuser juste avant l'administration.

**Revendications**

**1.** Composition médicamenteuse comprenant, en quantités thérapeutiquement efficaces,
du chlorure de sodium hypertonique,

au moins une molécule exerçant de manière directe ou indirecte un effet de relaxation des muscles lisses vasculaires, et
au moins une molécule susceptible de fournir un apport exogène en phosphates.

**2.** Composition suivant la revendication 1, **caractérisée en ce qu'**elle comprend en outre un agent alcalinisant susceptible d'augmenter un pH sanguin dans une gamme compatible avec un transport d'oxygène adéquat.

**3.** Composition suivant l'une ou l'autre des revendications 1 et 2, **caractérisée en ce qu'**elle se présente sous la forme d'au moins une solution aqueuse.

**4.** Composition suivant la revendication 3, **caractérisée en ce qu'**elle consiste en une combinaison d'une première solution de perfusion et d'une deuxième solution de perfusion,
la première solution de perfusion comprenant de l'eau, ledit chlorure de sodium hypertonique et ladite au moins une molécule exerçant un effet de relaxation, et
la deuxième solution de perfusion comprenant de l'eau, et ladite au moins une molécule susceptible de fournir un apport exogène en phosphates.

**5.** Composition suivant l'une ou l'autre des revendications 1 et 2, **caractérisée en ce qu'**elle se présente sous la forme d'au moins une formulation solide destinée à la constitution d'au moins une solution aqueuse de perfusion par mélange avec de l'eau.

**6.** Composition suivant la revendication 4, **caractérisée en ce que** le chlorure de sodium hypertonique est à une concentration de 5 à 10 % dans ladite première solution de perfusion.

**7.** Composition suivant l'une quelconque des revendications 1 à 6, **caractérisée en ce que** ladite au moins une molécule susceptible de fournir un apport exogène en phosphates est choisie parmi le groupe constitué des phosphates inorganiques, des phosphates organiques ou des modificateurs de la teneur intra-érythrocytaire en diphosphoglycérate.

8.  Composition suivant l'une quelconque des revendications 4 à 7, **caractérisée en ce que** la deuxième solution de perfusion contient en outre un agent alcalinisant susceptible d'augmenter le pH sanguin dans une gamme compatible avec un transport d'oxygène adéquat.

9.  Composition suivant l'une quelconque des revendications 2 à 8, **caractérisée en ce que** l'agent alcalinisant est choisi parmi le groupe constitué des bicarbonates, acétates, propionates et lactates.

10. Composition suivant l'une quelconque des revendications 4 à 8, **caractérisée en ce que** la deuxième solution de perfusion contient en outre au moins un composant destiné à corriger l'équilibre hydrique, électrolytique et énergétique.

11. Composition suivant la revendication 10, **caractérisée en ce que** ledit au moins un composant destiné à corriger l'équilibre est choisi parmi le groupe constitué du chlorure de sodium, du chlorure de potassium et du glucose.

12. Composition suivant l'une quelconque des revendications 1 à 11, pour la mise en oeuvre d'un traitement thérapeutique ou prophylactique des troubles hypoxiques des tissus, en particulier de ceux survenant au cours de pathologies toxi-infectieuses, chez les mammifères.

13. Composition suivant la revendication 12, pour la mise en oeuvre dans le traitement de la diarrhée néonatale et le choc endotoxinique chez les ruminants.

14. Composition suivant l'une quelconque des revendications 4 à 11, comme produit de combinaison de la première solution de perfusion et de la deuxième solution perfusion destiné à une utilisation simultanée, séparée ou étalée dans le temps de celles-ci dans un traitement thérapeutique ou prophylactique de troubles hypoxiques des tissus, en particulier de ceux survenant au cours de pathologies toxi-infectieuses, chez les mammifères.

15. Utilisation d'une composition suivant l'une quelconque des revendications 1 à 11, pour la fabrication d'un médicament destiné au traitement thérapeutique ou prophylactique de troubles hypoxiques des tissus, en particulier de ceux survenant au cours de pathologies toxi-infectieuses, chez les mammifères.

*Fig. 1*

EP 1 256 360 A1

$f = \bullet$
$y = 0,37936 - 1,0304x$   $R^2 = 0,266$
$r = 0,52 \ (p<0,05)$
$e = \blacktriangle$
$y = 0,17104 - 0,52968x$   $R^2 = 0,504$
$r = 0,71 \ (p<0,05)$

*Fig. 2*

Fig.3

EP 1 256 360 A1

Fig. 4

Fig. 5

P50  v(mm Hg)

Temps (h)

a = ×
b = +
c = ◖
d = ı
f = ●
e = ◆

EP 1 256 360 A1

14

*Fig. 6*

a ≒ ×
b = +
c = ❶
d = ❙
e = ◆
f = ●

Temps(h)

EP 1 256 360 A1

*Fig. 7*

*Fig. 8*

*Fig. 9*

**Office européen des brevets**

## RAPPORT PARTIEL DE RECHERCHE EUROPEENNE
qui selon la règle 45 de la Convention sur le brevet européen est considéré, aux fins de la procédure ultérieure, comme le rapport de la recherche européenne

Numéro de la demande

EP 01 20 1715

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 00 69427 A (ROMASCHIN ALEXANDER D ;WALKER PAUL MOORE) 23 novembre 2000 (2000-11-23) | 1,3,7-15 | A61P1/12 A61P1/00 A61P39/06 A61K33/42 |
| Y | * page 2, ligne 20 - page 3, ligne 25; revendications 1,6,8; exemple 1 * * page 6, ligne 21-24 * | 1,3, 12-15 | |
| X | US 5 686 488 A (NIXON JON C ET AL) 11 novembre 1997 (1997-11-11) * colonne 5, ligne 26-28; revendications 4,6,7; exemples 1,5,7,9 * * colonne 3, ligne 1,2 * * colonne 1, ligne 9-14 * | 1-4,6-15 | |
| X | US 5 654 266 A (CHEN CHUNG-HO ET AL) 5 août 1997 (1997-08-05) * colonne 3, ligne 2-4; exemple 1; tableau 2 * * colonne 7, ligne 61,62 * * colonne 8, ligne 3-8 * * revendications 1-3; tableau 3 * * colonne 6, ligne 59,60 * | 1-3,6-8, 10,11 | |

--/--

**DOMAINES TECHNIQUES RECHERCHES (Int.Cl.7)**

A61K

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| LA HAYE | 18 janvier 2002 | Kanbier, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03 82 (P04C09)

Office européen
des brevets

**RECHERCHE INCOMPLETE
FEUILLE SUPPLEMENTAIRE C**

Numéro de la demande

EP 01 20 1715

Revendications ayant fait
l'objet de recherches incomplètes:
       1-14

Raison pour la limitation de la recherche:

Les revendications 1-14 présentes ont trait à des compositions et leurs
utilisations, dans lesquelles il s'agit de composés défini en faisant
référence à une caractéristique ou propriété souhaitable, à savoir:
"molécule exerçant de manière ... vasculaires",
"molécule susceptible ... phosphates",
"modificateurs de ... diphosphoglycérate",
"agent 'alcalinisant! susceptible d'augmenter ... adéquat",
"molécule exerçant ... relaxation", et
"composant destiné ... énergétique".
Les revendications couvrent tous les composés présentant ces
caractéristiques ou propriétés, alors que la demande ne fournit un
fondement au sens de l'Article 84 CBE et/ou un exposé au sens de
l'Article 83 CBE que pour un nombre limité de tels composés. Dans le cas
présent, les revendications manquent de fondement et la demande manque
d'exposé à un point tel qu'une recherche significative sur tout le
spectre couvert par les revendications est impossible.
Indépendamment des raisons évoquées ci-dessus, les revendications
manquent aussi de clarté. En effet, il est impossible de décrire un
composé d'une façon suffisante par son mécanisme d'action et/ou par son
profil pharmacologique. Ce manque de clarté est, dans le cas présent, de
nouveau tel qu'une recherche significative sur tout le spectre couvert
par les revendications est impossible.

En outre, la revendication présente a trait à une très grande variété
d'utilisations par le terme "étalée dans le temps". Ceci résulte en
manque de clarté (et/ou de concision) au sens de l'Article 84 CBE qui
s'en suit d'une importance telle qu'une recherche significative de
l'objet total des revendications devient impossible.

Finalement, les revendications 7, 9 et 12-15 présentes ont trait à des
compositions et leurs utilisations, dans lesquelles il s'agit d'une très
grande variété de composés par l'utilisation dans la revendication 9 des
termes "bicarbonates", "acétates", "propionates", "lactates"; et
"phosphates inorganiques 'ou! phosphates organiques" (ce qui revient à
"phosphates" tout court).
Un fondement au sens de L'Article 84 CBE et/ou un exposé au sens de
l'Article 83 CBE ne peut cependant être trouvé que pour un nombre
restreint de ces composés revendiqués. Dans le cas présent, les
revendications manquent à un tel point de fondement et l'exposé de
l'invention dans la description est si limité qu'une recherche
significative couvrant tout le spectre revendiqué est impossible.

En conséquence, la recherche  n'a été effectuée que pour les parties des
revendications dont l'objet apparaît être clair (et/ou concis), fondé et
suffisamment exposé, à savoir les parties concernant les composés
spécifiques divulgués dans les revendications et les examples, en tenant
duement compte de la description de cette demande.

**Office européen
des brevets**

**RAPPORT PARTIEL
DE RECHERCHE EUROPEENNE**

Numero de la demande

EP 01 20 1715

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 0 719 559 A (CHEMEDICA SA) 3 juillet 1996 (1996-07-03) * revendication 13; exemple 1 * | 1,3,6,7, 10,11 | |
| A | K. PARFITT, ED.: "Martindale, the complete drug reference" 1999 , PHARMACEUTICAL PRESS , LONDON XP002187729 * page 1157, colonne du milieu – page 1159, colonne du milieu * * page 338, colonne de gauche * * page 796, colonne du milieu * | 1,15 | |
| Y A | FR 2 223 029 A (UNIV COLORADO RES) 25 octobre 1974 (1974-10-25) * page 1, ligne 31 – page 3, ligne 26; revendications 1,4-9 * * page 5, colonne 12-18 * * page 8, alinéa 2 * * page 11, ligne 18,19 * | 1,3, 12-15 2,5,8-11 | DOMAINES TECHNIQUES RECHERCHES (Int.Cl.7) |
| Y | US 6 015 835 A (MIYAMOTO KOHO ET AL) 18 janvier 2000 (2000-01-18) * colonne 1, ligne 17-27; revendications 1,6,7; exemples 2-5 * * colonne 12, ligne 59-61 * * colonne 14, ligne 37-39 * | 1,3,12, 14,15 | |

-/--

**Office européen**
**des brevets**

**RAPPORT PARTIEL**
**DE RECHERCHE EUROPEENNE**

Numero de la demande

EP 01 20 1715

## DOCUMENTS CONSIDERES COMME PERTINENTS

| Catégorie | Citation du document avec indication, en cas de besoin, des parties pertinentes | Revendication concernée |
|---|---|---|
| Y | DATABASE WPI<br>Week 200064<br>Derwent Publications Ltd., London, GB;<br>AN 2000-663143<br>XP002187730<br>& RU 2 150 871 A (CHUKHROVA M G),<br>20 juin 2000 (2000-06-20) | 1,3,12, 14,15 |
| A | * abrégé *<br>& RU 2 150 781 A (CHUKHROVA M G)<br>20 juin 2000 (2000-06-20)<br>* colonne 7; revendications 3-5; exemples;<br>tableau 1 *<br>& CHEMICAL ABSTRACTS, vol. 135, no.<br>210341,<br>Columbus, Ohio, US;<br>abstract no. 210341,<br>CHUKHROVA M G: "Alcohol-free beverage for removal of alcohol withdrawal syndrome"<br>* abrégé * | 6 |
| Y | US 5 296 241 A (KAMATH BURDE L ET AL)<br>22 mars 1994 (1994-03-22) | 1,3,12, 14,15 |
| A | * colonne 1, ligne 13-16; revendications 1,2,8 * | 5,8-11 |
| A | PATENT ABSTRACTS OF JAPAN<br>vol. 1997, no. 09,<br>30 septembre 1997 (1997-09-30)<br>& JP 09 124492 A (MATERIAL ENG TECH LAB INC), 13 mai 1997 (1997-05-13)<br>* abrégé *<br>& DATABASE WPI<br>Week 119729<br>Derwent Publications Ltd., London, GB;<br>AN 1997-316483<br>& JP 09 124492 A (SHINSOZAI SOGO KENKYUSHO KK), 13 mai 1997 (1997-05-13)<br>* abrégé * | 1-4,8,9, 12,14 |

-/--

**CLASSEMENT DE LA DEMANDE** (Int.Cl.7)

**DOMAINES TECHNIQUES RECHERCHES** (Int.Cl.7)

**EP 1 256 360 A1**

Office européen
des brevets

**RAPPORT PARTIEL
DE RECHERCHE EUROPEENNE**

Numero de la demande

EP 01 20 1715

| | DOCUMENTS CONSIDERES COMME PERTINENTS | | CLASSEMENT DE LA DEMANDE (Int.Cl.7) |
|---|---|---|---|
| Catégorie | Citation du document avec indication, en cas de besoin, des parties pertinentes | Revendication concernée | |
| A | EP 0 382 306 A (DUPHAR INT RES) 16 août 1990 (1990-08-16) * page 2, colonne 1-23; revendications; exemples * * page 3, ligne 20-23 * * page 5, ligne 20-30 * | 1-3,5, 8-13,15 | |
| A | DATABASE WPI Week 198551 Derwent Publications Ltd., London, GB; AN 1985-318682 XP002187731 & ES 8 504 625 A (FISONS IBERICA SA), 16 juillet 1985 (1985-07-16) * abrégé * | 1 | |

DOMAINES TECHNIQUES
RECHERCHES (Int.Cl.7)

EPO FORM 1503 03.82 (P04C11)

**ANNEXE AU RAPPORT DE RECHERCHE EUROPEENNE
RELATIF A LA DEMANDE DE BREVET EUROPEEN NO.**

EP 01 20 1715

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-01-2002

| Document brevet cité au rapport de recherche | | Date de publication | Membre(s) de la famille de brevet(s) | | Date de publication |
|---|---|---|---|---|---|
| WO 0069427 | A | 23-11-2000 | WO | 0069427 A1 | 23-11-2000 |
| US 5686488 | A | 11-11-1997 | AUCUN | | |
| US 5654266 | A | 05-08-1997 | US | 5298487 A | 29-03-1994 |
| EP 0719559 | A | 03-07-1996 | IT | 1274984 B | 29-07-1997 |
| | | | AT | 171623 T | 15-10-1998 |
| | | | CA | 2164770 A1 | 10-06-1996 |
| | | | DE | 69505095 D1 | 05-11-1998 |
| | | | DE | 69505095 T2 | 15-04-1999 |
| | | | DK | 719559 T3 | 21-06-1999 |
| | | | EP | 0719559 A1 | 03-07-1996 |
| | | | ES | 2124486 T3 | 01-02-1999 |
| | | | JP | 8253505 A | 01-10-1996 |
| | | | US | 5871772 A | 16-02-1999 |
| | | | ZA | 9510441 A | 19-06-1996 |
| FR 2223029 | A | 25-10-1974 | US | 3928574 A | 23-12-1975 |
| | | | AU | 6713174 A | 02-10-1975 |
| | | | BE | 812907 A1 | 27-09-1974 |
| | | | CA | 1027865 A1 | 14-03-1978 |
| | | | DE | 2414593 A1 | 17-10-1974 |
| | | | FR | 2223029 A1 | 25-10-1974 |
| | | | GB | 1466153 A | 02-03-1977 |
| | | | ZA | 7401978 A | 26-03-1975 |
| US 6015835 | A | 18-01-2000 | US | 6316501 B1 | 13-11-2001 |
| | | | AU | 703540 B2 | 25-03-1999 |
| | | | AU | 1639297 A | 09-10-1997 |
| | | | CA | 2200795 A1 | 26-09-1997 |
| | | | EP | 0797992 A2 | 01-10-1997 |
| | | | JP | 10045578 A | 17-02-1998 |
| RU 2150871 | A | | AUCUN | | |
| US 5296241 | A | 22-03-1994 | AUCUN | | |
| JP 09124492 | A | 13-05-1997 | AUCUN | | |
| EP 0382306 | A | 16-08-1990 | AU | 634746 B2 | 04-03-1993 |
| | | | AU | 4905190 A | 16-08-1990 |
| | | | CA | 2009168 A1 | 07-08-1990 |
| | | | EP | 0382306 A1 | 16-08-1990 |
| | | | IE | 81127 B1 | 22-03-2000 |
| | | | IL | 93284 A | 28-11-1994 |

Pour tout renseignement concernant cette annexe : voir Journal Officiel de l'Office européen des brevets, No. 12/82

## ANNEXE AU RAPPORT DE RECHERCHE EUROPEENNE
## RELATIF A LA DEMANDE DE BREVET EUROPEEN NO.

EP 01 20 1715

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

18–01–2002

| Document brevet cité au rapport de recherche | | Date de publication | Membre(s) de la famille de brevet(s) | | Date de publication |
|---|---|---|---|---|---|
| EP 0382306 | A | | JP | 2270822 A | 05–11–1990 |
| | | | NZ | 232346 A | 26–03–1992 |
| | | | US | 5164192 A | 17–11–1992 |
| | | | ZA | 9000810 A | 28–11–1990 |
| ES 8504625 | A | | AUCUN | | |

Pour tout renseignement concernant cette annexe : voir Journal Officiel de l'Office européen des brevets, No.12/82